# DEMANDE DE BREVET EUROPEEN

(11) **EP 0 811 357 A1**
(43) Date de publication de la demande: **10.12.1997**
(21) Numéro de dépôt: 97401214.8
(22) Date de dépôt: 02.06.1997
(51) Int. Cl.: A61B 17/70

(54) **Dispositif de liaison transversale rigide entre deux tiges d'ostéosynthèse du rachis**

(30) Priorité: 03.06.1996 FR 9606800
(71) Demandeur: Stryker France S.A., 93290 Tremblay-en-France (FR)
(72) Inventeur: Baccelli, Christian, 33650 St Medard d'Eyrans (FR); Le Couedic, Régis, 33160 St Medard en Jalles (FR); Petreto, Eric, 33610 Cestas (FR)
(74) Mandataire: Le Forestier, Eric

(57) **Abrégé**

Un dispositif de liaison transversale entre deux tiges longitudinale en ostéosynthèse du rachis comprend deux éléments de liaison et de blocage (30) comportant deux branches (33, 34) séparées par un canal central (35). Chaque branche forme un crochet définissant un berceau (33a, 34a) pour une tige (10a; 10b), et les berceaux sont tournés vers deux faces opposées de l'élément. Le dispositif comprend également un élément de liaison transversale (20) entre les deux éléments de liaison et de blocage (30).

Ainsi chaque élément peut être engagé sur une tige par réception de la tige associée dans son canal central, puis tourné sur lui-même pour ancrer la tige associée dans les berceaux des deux branches.

Selon l'invention, l'élément de liaison transversale consiste en une barre (20) qui peut être glissée longitudinalement dans les canaux une fois disposés sensiblement dans l'alignement l'un de l'autre, et il est prévu en outre un organe de blocage (40) coopère avec chaque élément de liaison et de blocage (30) pour serrer ladite barre (20) entre le fond (35a) du canal et la tige (10a; 10b) et assurer le blocage.

## Description

La présente invention a trait d'une façon générale aux dispositifs pour l'ostéosynthèse du rachis, et plus particulièrement un dispositif de liaison transversale rigide entre deux tiges d'ostéosynthèse s'étendant dans le sens longitudinal de la colonne vertébrale et ancrées sur les vertèbres.

On connaît déjà de tels dispositifs de liaison transversale.

On connaît en particulier par EP-A-0 446 092 un dispositif de liaison transversale dans lequel deux crochets aptes à s'engager sur deux tiges longitudinales coopèrent avec des vis de blocage et avec une barre transversale pour réaliser une liaison simple et pratique à mettre en oeuvre.

Toutefois le blocage des tiges dans les crochets peut s'avérer insuffisant dans le cas où le dispositif est soumis à des contraintes latérales importantes. Plus precisément, les tiges peuvent glisser hors des crochets et supprimer ainsi la liaison, ce qui est tout à fait indésirable.

On connaît également par US-A-5 334 203 un dispositif de liaison transversale tel que défini dans le préambule de la revendication 1.

Ce dipositif connu comprend en outre, pour assurer le blocage de l'ensemble, un dispositif mécanique relativement complexe, et comportant de nombreuses pièces différentes. Ceci est désavantageux en ce que la pose du dispositif est extrêmement fastidieuse, notamment du fait de la manipulation nécessaire de ce grand nombre de pièces.

En outre, un inconvénient majeur de ce dispositif connu réside en ce qu'il est nécessairement pré-assemblé avant la pose, et qu'il présente alors un encombrement très important alors qu'il doit être rapporté sur les deux tiges longitudinales par un déplacement comportant une composante substantielle dans une direction perpendiculaire au plan passant par les deux tiges.

Il en résulte la nécessité d'une destruction importante de la structure ligamentaire s'étendant le long de la colonne vertébrale entre les deux tiges.

Par ailleurs, les nombreux degrés de liberté du dispositif pré-assemblé, nécessaires pour s'adapter à la configuration d'espèce des tiges, peuvent rendre son montage peu pratique.

Enfin et surtout, ce document indique que les éléments de liaison et de blocage doivent être préalablement montés à rotation sur l'élément de liaison transversale pour que le dispositif puisse être commodément utilisé.

La présente invention est basée sur la découverte du fait que des éléments de liaison et de blocage du genre défini ci-dessus pourraient permettre, sans être montés à rotation sur d'autres parties du dispositif, d'améliorer les qualités d'un dispositif à simple barre transversale tel que décrit dans EP-A-0 446 092.

Plus précisément, il a été découvert que l'on pouvait monter préalablement les éléments de liaison et de blocage sur la tige, et les faire tourner sur eux-mêmes avant l'assemblage de l'implant, pour pouvoir ensuite utiliser une simple barre, glissée dans ces éléments, comme moyen de liaison transversale.

La présente invention vise ainsi à pallier les inconvénients de l'état de la technique, et à proposer un dispositif de liaison transversale qui conserve la facilité d'utilisation et l'efficacité des éléments de liaison et de blocage de ce document, tout en présentant la simplicité de structure du dispositif de EP-A-0 446 092

Elle propose à cet effet un dispositif tel que défini dans la revendication 1.

Des aspects préférés, mais non limitatifs de ce dispositif sont définis dans les sous-revendications.

D'autres aspects, buts et avantages de la présente invention apparaîtront mieux à la lecture de la description détaillée suivante d'une forme de réalisation préférée de celle-ci, donnée à titre d'exemple et faite en référence aux dessins annexés, sur lesquels :
la figure 1 est une vue de profil et partiellement en coupe d'un dispositif de liaison transversale selon l'invention,
la figure 2 est une vue postérieure du dispositif de la figure 1,
la figure 3 est une vue en perspective éclatée de trois composants du dispositif des figures 1 et 2, et
les figures 4a et 4b, 5a et 5b, et 6a et 6b, sont des vues partielles respectivement de profil et de l'arrière illustrant la pose du dispositif de liaison des figures 1 et 2.

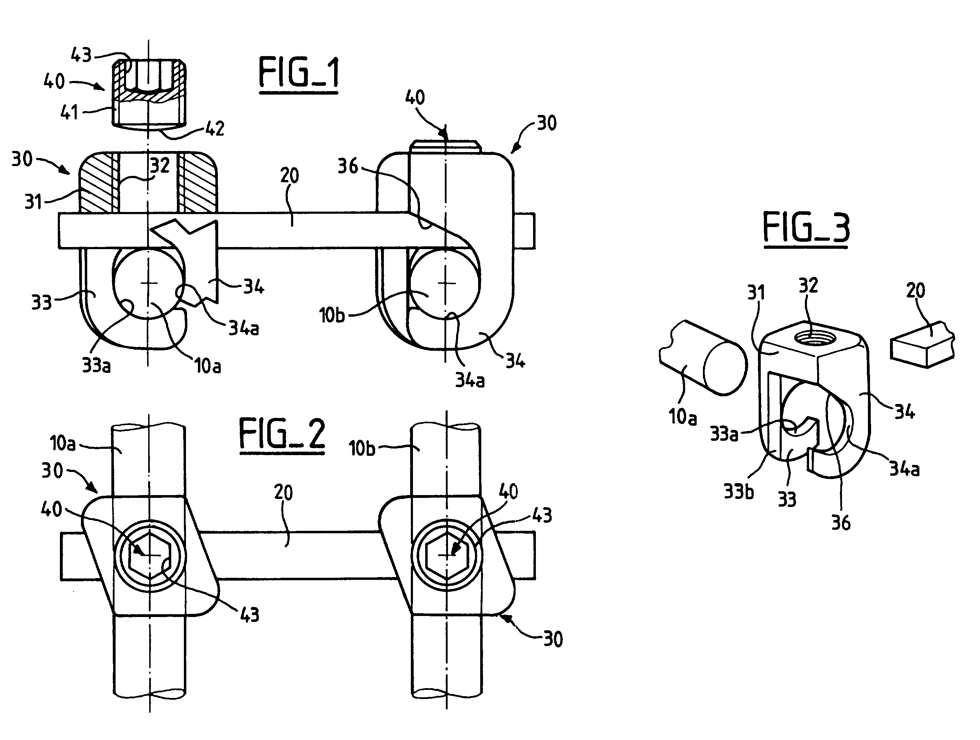

En référence aux figures 1 et 2, on a représenté deux tiges d'union longitudinale entre vertèbres, respectivement 10a et 10b, illustrées comme étant parallèles l'une à l'autre mais pouvant, dans la pratique, prendre diverses inclinaisons mutuelles dans des gammes angulaires limitées.

Ces deux tiges, de façon conventionnelle, sont cylindriques et lisses. Elle sont ancrées de place en place sur les vertèbres, à l'aide de dispositifs bien connus.

Le dispositif de liaison transversale selon l'invention comprend une barre transversale 20, en l'espèce de section rectangulaire, et deux éléments de liaison et de blocage 30 identiques, aptes à recevoir des vis de blocage 40.

Plus précisément, chaque élément 30 comprend une base 31 en forme générale de losange, et deux branches 33, 34 s'étendant parallèlement l'une à l'autre à partir de la base 31, au niveau de deux côtés opposés de celle-ci.

Chaque branche présente la forme générale d'un "J", formant crochet, et définit sur son bord intérieur en creux un berceau, respectivement 33a, 34a. Les deux berceaux s'ouvrent selon deux directions opposées. Chaque berceau est surplombé par une surface oblique 36 qui est montante, c'est-à-dire s'éloigne du plan défini par le fond des berceaux, de l'intérieur vers l'extérieur. Cette surface est destinée, comme on le verra plus loin, à former une came sollicitant la tige associée 10a ou 10b vers le fond dudit berceau lorsque l'élément est tourné sur lui-même, de manière à faciliter la pose.

Les deux branches définissent entre elles un canal droit 35 dont le fond 35a est défini par la face plane intérieure de la base 31.

Ce canal est défini par deux faces internes principales 33c, 34c des deux branches, et par deux faces biseautées 33b, 34b adjacentes auxdites faces principales et situées au niveau des deux débouchés opposés du canal, immédiatement en arrière du berceau respectif comme le montrent en particulier les figures 3, 4b, 5b et 6b.

Cette disposition des faces internes des branches 33, 34 permet de délimiter, entre les faces 33c, 34c, un chemin adapté à la tige respective 10a ou 10b, tout en autorisant, grâce aux parois en biseau 33b, 34b, le passage de la barre 20 après rotation sur eux-mêmes des éléments 30 selon un angle α (voir figure 5b) sensiblement inférieur à 90°.

On observera ici que la largeur du canal 35 entre les faces 33c, 34c est choisie légèrement supérieure au diamètre de la tige 10a ou 10b, tandis que la largeur du canal vis-a-vis de la barre 20 mise en place perpendiculairement à la tige est également légèrement supérieure à la largeur de ladite barre, de manière à autoriser un certain débattement angulaire entre l'élément 30 et la barre 20.

Enfin un trou taraudé traversant 32 s'étend dans la base 31, entre sa face extérieure et le fond 35a.

Une vis de blocage 40 est destinée à être vissée dans le trou taraudé 32. Elle comporte un corps cylindrique autour duquel est formé un filetage 41, un sommet 42 de forme légèrement bombée, et une empreinte 43 pour outil de vissage, par exemple une empreinte standard à six pans en creux.

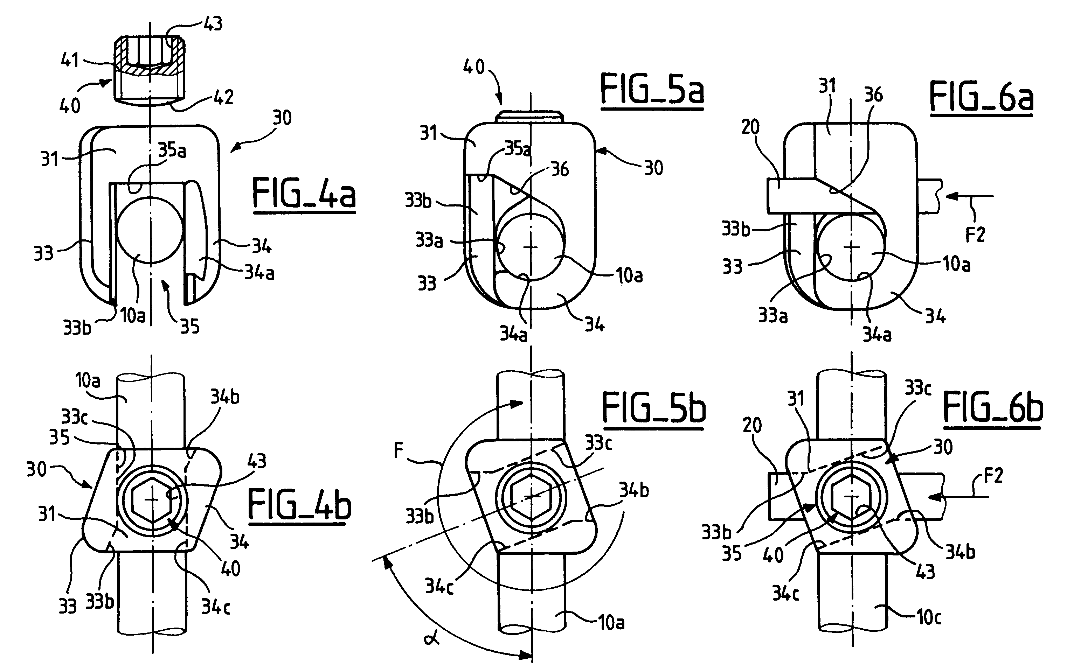

On va maintenant décrire en référence aux figures 4a, 4b, 5a, 5b, 6a et 6b le processus de pose du dispositif décrit ci-dessus.

On place tout d'abord l'un des deux éléments 30 l'une des deux tiges 10a (ou 10b), sensiblement à la même hauteur, de telle sorte que la tige soit reçue dans le canal 35 (figures 4a et 4b). A ce stade, la vis 40 peut être pré-montée dans son trou taraudé 32.

Ensuite, l'élément 30 est tourné sur lui-même (flèche F1) sur l'angle α, en l'espèce égal à 70°, jusqu'à une position de butée définie par la venue en contact de la tige avec le fond des berceaux 33a, 34a des deux branches 33, 34 (figures 5a et 5b). On observera qu'au cours de ce mouvement, c'est la surface supérieure oblique 36 de l'élément 30 qui joue le rôle d'une came qui repousse la tige 10a vers le fond des berceaux 33a, 34a.

La barre transversale 20 est alors introduite par l'un ou l'autre des deux côtés de l'élément 30, par exemple selon la flèche F2 des figures 6a et 6b, de manière à s'engager dans le canal 35 de l'élément 30 entre la tige 10a et le fond 35a dudit canal. On notera que la barre 20 est alors positionnée de manière à laisser la région de l'autre tige 10b dégagée pour la pose subséquente de l'autre élément 30.

Cet autre élément 30 est alors mis en place, par translation puis rotation comme clans le cas du premier élément, sur sa tige 10b.

Enfin on fait coulisser la barre 20 de manière à ce qu'elle s'engage dans ledit autre élément 30 de la même manière que pour le premier élément, c'est-à-dire entre la tige 10b et le fond du canal associé.

Les deux vis 40 sont alors serrées à l'aide d'un outil, de manière à bloquer ladite barre 0 entre les fonds 35a des canaux et les tiges, la force de réaction nécessaire étant excercée sur les tiges par les parties d'extrémité libre courbes des branches 33, 34.

Ce blocage assure en même temps le blocage mutuel des éléments et des tiges, si bien que l'on obtient, par une procédure de montage extrêmement simple et peu contraignante pour les tissus environnants, une liaison transversale rigide extrêmement efficace.

On notera ici que l'épaisseur de la barre transversale 40 est choisie de telle sorte que cette barre puisse, au serrage, subir un certain degré de cintrage. Ceci permet au dispositif de faire face à des situations dans lesquelles les deux tiges 10a, 10b ne se trouvent pas dans le même plan. En outre, comme on l'a indiqué plus haut, le débattement angulaire de la barre 20 dans les canaux 35 permet de faire face à des situations dans lesquelles les deux barres divergent ou convergent légèrement en projection dans le plan transversal.

On notera à cet égard que la face d'appui bombée 42 de chaque vis 40 permet de faire face à des positions mutuelles différentes sans compromettre la qualité du blocage.

On observera par ailleurs que le dispositif selon l'invention permet d'effectuer une liaison rigide entre les tiges sans qu'un effort de traction transversale indésirable soit exercé sur lesdites tiges.

Naturellement, toutes les pièces consitutives du dispositif selon l'invention sont réalisées en un matériau bio-compatible tel que l'acier inoxydable ou un alliage de titane. En outre, la forme et la dimension des pièces, et notamment la longueur de la barre 20, peuvent être choisies en fonction des besoins.

Bien entendu, la présente invention n'est nullement limitée à la forme de réalisation décrite ci-dessus et représentée sur les dessins, mais l'homme du métier saura y apporter toute variante ou modification conforme à son esprit.

## Revendications

1. Dispositif de liaison transversale entre deux tiges longitudinales en ostéosynthèse du rachis, du type comprenant deux éléments de liaison et de blocage (30) comprenant deux branches (33, 34) séparées par un canal central (35), chaque branche présentant la forme d'un crochet définissant un berceau (33a, 34a) pour une tige longitudinale (10a; 10b), et les deux berceaux étant tournés respectivement vers deux faces opposées de l'élément de liaison et de blocage, de telle sorte que chaque élément de liaison et de blocage peut être engagé sur une tige par réception de la tige associée dans son canal central, et peut être ensuite tourné sur lui-même pour ancrer la tige associée dans les berceaux des deux branches, le dispositif comprenant en outre un élément de liaison transversale (20) entre les deux éléments de liaison et de blocage (30), dispositif caractérisé en ce que l'élément de liaison transversale est constitué par une barre (20) apte à être glissée longitudinalement dans les canaux (35) des deux éléments de liaison et de blocage une fois disposés sensiblement dans l'alignement l'un de l'autre, et en ce qu'il est prévu en outre un organe de blocage (40) apte à coopérer avec chaque élément de liaison et de blocage (30) et à serrer ladite barre (20) entre le fond (35a) du canal et la tige (10a; 10b) pour assurer le blocage de l'ensemble.

2. Dispositif selon la revendication 1, caractérisé en ce que chaque élément de liaison et de blocage (30) comprend une base (31) réunissant lesdites branches et définissant le fond (35a) du canal (35), en ce qu'un trou taraudé (32) est formé dans ladite base, et en ce que l'organe de blocage associé est constitué par une vis (40) engagée dans ledit trou taraudé.

3. Dispositif selon la revendication 2, caractérisé en ce que ladite vis (40) possède une extrémité (42) d'appui sur la barre qui est légèrement convexe.

4. Dispositif selon l'une des revendications 1 à 3, caractérisé en ce que ladite barre (20) présente une section polygonale, en particulier rectangulaire.

5. Dispositif selon l'une des revendications 1 à 4, caractérisé en ce que ladite barre (20) présente une largeur légèrement inférieure à la largeur de chaque canal (35) dans la région de son fond (35a), de manière à autoriser de légères inclinaisons mutuelles des deux éléments de liaison et de blocage.

6. Dispositif selon l'une des revendications 1 à 5, caractérisé en ce que, dans chaque élément de liaison et de blocage (30), l'angle (α) formé entre la direction longitudinale du canal (35) et la direction longitudinale du trajet de tige défini par les deux berceaux (33a, 34a) est inférieur à 90°.

7. Dispositif selon la revendication 6, caractérisé en ce que ledit angle (α) est compris entre environ 65 et 75°.

8. Dispositif selon l'une des revendications 6 et 7, caractérisé en ce que le canal (35) est défini par des faces internes desdites branches comprenant chacune une face principale (33c, 34c) destinée à guider la tige et une face d'extremité en biseau (33b, 34b) destinée à guider la barre.

9. Dispositif selon l'une des revendications 1 à 8, caractérisé en ce que chaque berceau (33a, 34a) de chaque élément de liaison et de blocage (30) est surplombé par une surface oblique (36) apte à solliciter la tige associée (10a, 10b) vers le fond dudit berceau lorsque l'élément de liaison et de blocage est tourné sur lui-même.
